# EUROPÄISCHE PATENTSCHRIFT

(11) **EP 3 320 924 B1**
(45) Veröffentlichungstag und Bekanntmachung des Hinweises auf die Patenterteilung: **25.12.2019**
(21) Anmeldenummer: 17196738.3
(22) Anmeldetag: 17.10.2017
(51) Int. Cl.: A61L 24/06, A61L 24/00, A61L 27/16, A61L 27/50

(54) **VERFAHREN ZUR HERSTELLUNG EINES POLYMETHYLMETHACRYLAT-KNOCHENZEMENTS**
METHOD FOR PRODUCING A POLYMETHYL METHACRYLATE BONE CEMENT
PROCÉDÉ DE FABRICATION D'UN CIMENT OSSEUX DE POLYMÉTHACRYLATE DE MÉTHYLE

(30) Priorität: 11.11.2016 DE 102016222158
(43) Veröffentlichungstag der Anmeldung: 16.05.2018
(60) Teilanmeldung: 18203318.3
(73) Patentinhaber: Heraeus Medical GmbH, 61273 Wehrheim (DE)
(72) Erfinder: Kluge, Thomas, 56179 Vallendar (DE); Vogt, Sebastian, 99092 Erfurt (DE)
(74) Vertreter: Heraeus IP

(56) Entgegenhaltungen:
- EP-A1- 2 402 041
- EP-A2- 2 687 239
- DE-A1-102014 109 234

## Beschreibung

Gegenstand der Erfindung ist ein Verfahren zur Herstellung eines Polymethylmethacrylat-Knochenzement.

Polymethylmethacrylat-(PMMA)-Knochenzemente gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs, bis dieser erstarrt.

Polymethylmethacrylat-Knochenzemente können in geeigneten Mischbechern mit Hilfe von Spateln durch manuelles Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepack-Mischsysteme wurden in den Patenten EP 0 692 229 A1, DE 10 2009 031178 B3, US 5,997,544 A, WO 00/35506 A1 und US 5,588,745 A beschrieben.

Bei der Verwendung fast aller bisher bekannten Full-Prepack-Mischsysteme muss der medizinische Anwender die Zementkomponenten, das Zementpulver und die Monomerflüssigkeit manuell durch Betätigung von Mischvorrichtungen, wie Mischstäben mit Rührflügeln, zu einem Zementteig vermischen. Die Homogenität des gebildeten Zementteigs hängt wesentlich von der Durchführung der manuellen Vermischung ab und kann daher Schwankungen unterliegen.

Mischverfahren und Einrichtungen, die kein manuellen Mischen der Komponenten erfordern, wie sie in WO 00/35506 A1 und US 5,588,745 A beschrieben sind, erfordern kompliziert aufgebaute Einrichtungen und/oder funktionieren häufig nur unter speziellen Voraussetzungen.

So wurde in WO 00/35506 A1 zur Vermeidung von möglichen Inhomogenitäten des Zementteigs eine Lagerungs- und Mischvorrichtung vorgeschlagen, bei der die Zementkomponenten ohne manuell angetriebene mechanische Vermischung miteinander vermischt werden. Das Polymethylmethacrylat-Knochenzementpulver wird dabei in einer Kartusche gelagert, wobei das Zementpulver das gesamte Volumen der Kartusche ausfüllt und die Zwischenräume zwischen den Partikeln des Zementpulvers ein solches Volumen haben, das dem Volumen der Monomerflüssigkeit entspricht, die zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird und durch Vakuum, das an einem Vakuumanschluss an der Unterseite der Kartusche anliegt, durch das Zementpulver gezogen wird, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet. Nachteilig an diesem System ist, dass alle bisher auf dem Markt verfügbaren Zementpulver mit dieser Vorrichtung nicht angemischt werden können, weil die schnell anquellenden Zementpulverpartikel nach einem Eindringen der Monomerflüssigkeit in das Zementpulver in eine Tiefe von ungefähr 1-2 cm eine gelartige Barriere bilden und die weitere Migration der Monomerflüssigkeit durch das gesamte Zementpulver behindern. Konventionelle Zementpulver zeigen zudem das Phänomen, dass auf Grund der unterschiedlichen Oberflächenenergien die Pulverpartikel durch Methylmethacrylat nur schlecht benetzt werden. Dadurch dringt das Methylmethacrylat nur relativ langsam in das Zementpulver ein.

EP 2 402 041 A1 offenbart eine Knochenzementzusammensetzung, einen Kit und ein Herstellungsverfahren für einen Knochenzement. Die Knochenzementzusammensetzung enthält Methacrylatpolymerteilchen mit großem Durchmesser von einem durchschnittlichen Teilchendurchmesser von 10 bis 60 µm, Methacrylatpolymerteilchen mit kleinem Durchmesser von einem durchschnittlichen Teilchendurchmesser von 0,1 bis 2,0 µm, ein Methacrylatmonomer und einen Polymerisationsinitiator.

DE 10 2014 109 234 A1 offenbart ein autopolymerisierbares 2-Komponenten-Prothesenbasismaterial und ein Verfahren zu seiner Herstellung umfassend (A) mindestens eine flüssige Monomerkomponente, und (B) mindestens eine pulverförmige Komponente, wobei das Prothesenmaterial in den Komponenten (A) und/oder (B), (i) mindestens einen Initiator oder ein Initiatorsystem für die Autopolymerisation, (ii) durch eine elastische Phase modifizierte Kern-Schale Partikel und (iii) mindestens ein Urethandimethacrylat enthält.

EP 2 687 239 A2 offenbart eine Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff.

Die Aufgabe der Erfindung besteht somit in der Entwicklung eines einfachen und unkomplizierten Verfahrens zum Anmischen eines Polymethylmethacrylat-Knochenzements, das keine komplizierten Vorrichtungen benötigt, unabhängig von der Bedienperson reproduzierbar ist und schnell einen applizierbaren Knochenzement bereitstellt.

Die Aufgabe der Erfindung wurde daher mit einem Verfahren zur Herstellung eines Polymethylmethacrylat-Knochenzements gelöst, mit den Schritten:
a) Bereitstellen eines Zementpulvers, das mindestens ein partikuläres Polymethylmethacrylat oder Polymethylmethacrylat-Copolymer der Siebfraktion kleiner 100 µm, einen Initiator, und mindestens ein in Methylmethacrylat unlösliches, partikuläres oder faserförmiges Additiv enthält, wobei das Additiv ein Aufsaugvermögen größer gleich 0,6 g Methylmethacrylat pro Gramm Additiv bei Raumtemperatur besitzt,
b) Bereitstellen einer Monomerlösung, die mindestens ein Methylmethacrylat und einen Aktivator enthält, und anschließendes In-Kontakt-Bringen des Zementpulvers und der Monomerlösung ohne Anwendung von Scherkräften.

Ferner kann die Aufgabe durch ein Knochenzement-Kit zur Verwendung in einem solchen Verfahren gelöst werden, welcher nicht Teil der beanspruchten Erfindung ist. Das Knochenzement-Kit umfasst a) ein Zementpulver, das mindestens ein partikuläres Polymethylmethacrylat oder Polymethylmethacrylat-Copolymer der Siebfraktion kleiner 100 µm, einen Initiator, und mindestens ein in Methylmethacrylat unlöslichen, partikulären oder faserförmigen Additiv, wobei das Additiv ein Aufsaugvermögen größer gleich 0,6 g Methylmethacrylat pro Gramm Additiv bei Raumtemperatur besitzt, und b) eine Monomerlösung, die mindestens ein Methylmethacrylat und einen Aktivator enthält.

Die Aufgabe kann auch durch einen nach obigem Verfahren hergestellten Kochenzement gelöst werden, welcher nicht Teil der beanspruchten Erfindung ist.

Es wurde überraschend gefunden, dass es möglich ist, durch einfaches In-Kontakt-Bringen eines im folgenden definierten Zementpulvers mit einer nachstehend definierten Monomerflüssigkeit einen klebfreien, plastisch verformbaren Knochenzementteig herzustellen, der selbstständig durch radikalische Polymerisation aushärtet, ohne dass es notwendig ist, den Zementteig manuell oder mit Hilfe von technischen Hilfsmitteln zu vermischen.

Die Erfindung beruht auf der überraschenden Beobachtung, dass durch Zusatz eines in Methylmethacrylat unlöslichen, partikulären oder faserförmigen Additivs, das ein Aufsaugvermögen von größer 0,6 g Methylmethacrylat pro Gramm Additiv bei Raumtemperatur besitzt, zu einem Zementpulver eines niedrig-viskosen Zements, ein modifiziertes Zementpulver erhalten wird, in das Monomerflüssigkeit über eine Strecke von mindestens 5 cm eingepresst werden kann. Das Additiv verbessert überraschend auch die Benetzung des Zementpulvers mit Monomerflüssigkeit. Es erleichtert das Eindringen der Monomerflüssigkeit in das Zementpulver. Das Additiv hat dabei einen "Docht-Effekt". Das Additiv leitet schon in sehr geringen Mengen ab 0,1 Gew.-% die Monomerflüssigkeit in das Innere des Zementpulvers. Weiterhin verzögert das Additiv das Verkleben der Polymerpartikel, wodurch die Ausbildung einer blockierenden Gelschicht verzögert wird und das Eindringen der Monomerflüssigkeit in das Zementpulver begünstigt wird.

Weiterhin wurde überraschend gefunden, dass durch den Zusatz des Additivs zu einem Zementpulver auch durch Vakuumeinwirkung Monomerflüssigkeit über eine Strecke von mindestens 3,0 cm ausgehend vom Einleitungspunkt der Monomerflüssigkeit in einer Vorrichtung gemäß WO 00/35506 A1 gesaugt werden kann. Es wurde weiterhin gefunden, dass durch Einpressen von Monomerflüssigkeit in das erfindungsgemäße Zementpulver ein homogener Zementteig entsteht, so dass eine mechanische Vermischung der Zementkomponenten nicht notwendig ist. Auf diese Weise entsteht nach Vermischung des Zementpulvers mit der Monomerflüssigkeit sofort ein klebfreier, plastisch verformbarer Zementteig, der verarbeitbar und applizierbar ist und selbstständig durch radikalische Polymerisation aushärtet.

Erfindungsgemäß wird ein Verfahren bereitgestellt, bei dem Zementpulver und Monomerflüssigkeit vermengt werden, indem die Monomerflüssigkeit in das Zementpulver eindringt, bzw. vom Zementpulver absorbiert wird. Dadurch ist kein mechanisches Mischen, sei es manuell oder durch technische Hilfsmittel notwendig und die Mischung kann ohne Anwendung von Scherkräften erfolgen.

Bevorzugt werden Monomerflüssigkeit und Zementpulver unmittelbar vor und während der Absorption so angeordnet, dass die Monomerflüssigkeit in Bezug auf die Erdoberfläche unterhalb des Zementpulvers angeordnet ist. Dadurch können im Zementpulver enthaltene Lufteinschlüsse auf einfache Weise entweichen.

Die Ausdrücke "enthalten" und "umfassen", wie sie hier in der vorliegenden Erfindung verwendet werden, schließen auch "bestehen aus" und "im Wesentlichen bestehen aus" mit ein.

Unter Raumtemperatur wird erfindungsgemäß eine Temperatur von 23°C verstanden.

Die Monomerflüssigkeit umfasst Methylmethacrylat als Monomer.

Geeignete Polymere in dem Zementpulver sind Polymethylmethacrylat und Copolymere von Methylmethacrylat mit einem oder mehreren damit copolymerisierbaren Monomeren, wie Methylacrylat, Styren und Ethylacrylat, welche als Pulver vorliegen.

Das Polymerpulver ist ein Polymerpulver der Siebfraktion < 100 µm. Damit enthält das Polymer ausschließlich Partikel, die durch ein Sieb mit einer Maschenweite von 100 µm fallen.

Es kann vorgesehen sind, dass die Zwischenräume im Zementpulver zwischen 25 Volumenprozent und 40 Volumenprozent liegen.

Der Gewichtsanteil des Polymers der Siebfraktion < 100 µm kann in breiten Bereichen variieren, wobei der Gewichtsanteil in dem Zementpulver bevorzugt im Bereich von 69,5-99,3 liegt.

Das Zementpulver enthält außerdem einen Polymerisationsinitiator. Bei dem Polymerisationsinitiator handelt es sich vorzugsweise um einen aktivierbaren Polymerisationsinitiator, z.B. ein Peroxid.

Peroxide sind bevorzugt. Das Peroxid weist vorzugsweise keine freien aciden Gruppen auf. Bei dem Peroxid kann es sich um ein organisches Peroxid oder ein anorganisches Peroxid handein, wie z.B. Dialkylperoxide oder Hydroperoxide. Beispielsweise kann das Peroxid aus der Gruppe ausgewählt sein, die aus Dibenzoylperoxid, Cumol-hydroperoxid, 1,1,3,3-Tetramethylbutylperoxid, t-Butylperoxid, t-Amyl-hydroperoxid, Di-Isopropylbenzen-monohydroperoxid und einer Mischung aus mindestens zwei davon besteht. Gemäß einer besonders bevorzugten Ausführungsform ist das Peroxid aus der Gruppe ausgewählt, die aus Dibenzoylperoxid und Dilauroylperoxid besteht. Besonders bevorzugt ist mit Wasser phlegmatisiertes Dibenzoylperoxid mit einem Wassergehalt von weniger als 30 Gew.-%, bevorzugt weniger als 28 Gew.-%.

Der Initiator wird dem Zementpulver in einem Anteil von 0,4-3,0 Gew.-%, bezogen auf das Zementpulver zugegeben.

Das Zementpulver enthält ferner ein in Methylmethacrylat unlösliches, partikuläres oder faserförmiges Additiv, wobei das Additiv ein Aufsaugvermögen größer gleich 0,6 g Methylmethacrylat pro Gramm Additiv bei Raumtemperatur besitzt,

Zur Bestimmung des Aufsaugvermögens der Additive wurde die aus der Pharmazie bekannte Enslin-Apparatur (C.-D. Herzfeldt, J. Kreuter (Hrsg.): Grundlagen der Arzneiformenlehre. Galenik 2, Springer Verlag Berlin Heidelberg New York, 1999, S. 79-80.) vereinfacht. Es wurde ein Glasfiltertiegel 1D3 der Firma Schott verwendet. Es wurde zuerst die Taramasse des Glasfiltertiegels bestimmt. Dann wurden jeweils 3,000 g bzw. 1,000 g des Additivs in den Glasfiltertiegel eingewogen. Der Glasfiltertiegel wurde auf eine Saugflasche aufgesetzt. Zum Additiv wurden 20 ml Methylmethacrylat gegeben, so dass das Additiv vollständig bedeckt war. Das nicht vom Additiv aufgesaugte Methylmethacrylat lief durch den Glasfiltertiegel nach unten ab. Nach 15 Minuten wurde der Glasfiltertiegel mit dem Additiv und dem aufgesaugten Methylmethacrylat ausgewogen und die Masse des aufgesaugten Methylmethacrylats bestimmt. Die Bestimmung wurde jeweils dreimal wiederholt und der Mittelwert bestimmt. Als Referenz wurde der Glasfiltertiegel mit Methylmethacrylat ohne zugesetztes Additiv in gleicher Weise behandelt.

Vorteilhaft ist es, wenn das Additiv kovalent gebundene Hydroxylgruppen an seiner Oberfläche besitzt. Besonders vorteilhaft sind dabei besonders Si-OH-Gruppen und alkoholische OH-Gruppen. Durch die oberflächlich angeordneten OH-Gruppen hat das Additiv eine hohe Oberflächenenergie, wodurch eine gute Benetzbarkeit des Additivs mit Methylmethacrylat gegeben ist.

Erfindungsgemäß sind als Additiv mikrokristalline Cellulose, Cellulose, Oxycellulose, Stärke, Titandioxid und Siliziumdioxid bevorzugt. Daneben kommen auch Zuckeralkohole wie Sorbitol, Mannitol, Dianhydroglucitol und Xylit als Additive in Betracht.

In einer Ausführungsform wird als Additiv pyrogenes Siliziumdioxid besonders bevorzugt. Die pyrogenen Kieselsäuren Aerosil® 380 und Aerosil® 300 sind besonders geeignet. Daneben ist es auch möglich, durch Sol-Gel-Prozesse erzeugtes Siliziumdioxid als Additiv zu verwenden.

Bevorzugt hat das Additiv eine Partikelgröße der Siebfraktion kleiner 100 µm, bevorzugt der Siebfraktion kleiner 50 µm und ganz besonders bevorzugt der Siebfraktion kleiner 10 µm.

Dem Knochenzementpulver kann wenigstens ein Röntgenopaker zugemischt werden. Beim Röntgenopaker kann es sich um einen fachüblichen Röntgenopaker handeln, bevorzugt in partikulärer Form. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen, wie Calciumcarbonat und Calciumsulfat besteht. Dabei sind Zirkoniumdioxid, Bariumsulfat, Calciumcarbonat und Calciumsulfat bevorzugt. Diese Röntgenopaker weisen vorzugsweise einen mittleren Teilchendurchmesser im Bereich von 10 nm bis 500 µm auf. Die Konzentration an zugemischtem Röntgenopaker, insbesondere die Konzentration an Zirkoniumdioxid, in dem Knochenzementpulver liegt bevorzugt in einem Bereich von 0 bis 30 Gew.-%, besonders bevorzugt in einem Bereich von 0 bis 15,0 Gew.-%.

In dem trockenen Zementpulver lassen sich pharmazeutische Wirkstoffe, wie Antiphlogistika, Bisphosphonate, Wachstumsfaktoren und bevorzugt Antiinfektiva und/oder Antiseptika relativ problemlos über einen längeren Zeitraum lagern. Als Antiinfektiva sind insbesondere Gentamicin, Tobramycin, Clindamycin, Vancomycin, Fosfomycin, Colistin und Daptomycin bevorzugt, die in Form von in Wasser leicht löslichen Salzen oder auch in Form von in Wasser gering löslichen Salzen oder Komplexen eingesetzt werden können. Als Antiseptika sind Octenidin, Dequaliniumchlorid, Polyhexanid, Calciumperoxid, und Benzalkoniumchlorid bevorzugt.

Die Monomerflüssigkeit enthält einen Aktivator. Aktivatoren aus der Gruppe der aromatischen Amine sind bevorzugt. Als Aktivator sind N,N-Di-methyl-p-toluidin, N,N-Di-methyl-o-toluidin, N,N-Dimethyl-anilin, N,N-Bis-hydroxyethyl-p-toluidin besonders bevorzugt.

Die Monomerflüssigkeit enthält bevorzugt auch einen radikalischen Stabilisator aus der Gruppe der Chinone oder der sterisch gehinderten Phenole. Als radikalischer Stabilisator werden p-Hydrochinon, o-Hydrochinon, 2,6-Di-t-butyl-p-hydrochinon, 2-t-butyl-p-hydrochinon und 2,6-Di-t-butyl-4-methyl-phenol bevorzugt.

Die Monomerflüssigkeit kann ferner eine farbgebende Substanz enthalten. Bei der farbgebenden Substanz handelt es sich z.B. um Farbpigmente, Lebensmittelfarbstoffe oder Farblacke. Beispiele sind E101, E104, E132, E141 (Chlorophyllin), E142, Riboflavin, Lissamingrün und Farblack Grün, das Aluminiumsalz einer Mischung aus E104 und E132. Chlorophyllin E141 ist bevorzugt.

Bevorzugt sind die Antiinfektiva und Antiseptika in dem Zementpulver in einem Anteil von 1,0 bis 10 Gew.-% enthalten.

In einer ersten Ausführungsform ist das Zementpulver aus 0,0-15,0 Gew.-% Röntgenopaker, 0,4-3,0 Gew.-% Dibenzoylperoxid, 79,5-99,3 Gew.-% Polymethylmethacrylat und/oder Polymethylmethacrylat-Copolymer, 0,1-2,5 Gew.-% Additiv zusammengesetzt.

In einer zweiten Ausführungsform ist das Zementpulver aus 1,0-10 Gew.-% eines Antiinfektivums oder Antiseptikums, 0,0-15,0 Gew.-% Röntgenopaker, 0,4-3,0 Gew.-% Dibenzoylperoxid, 69,5-98,3 Gew.-% Polymethylmethacrylat und/oder Polymethylmethacrylat-Copolymer, 0,1-2,5 Gew.-% Additiv zusammengesetzt.

Der durch das erfindungsgemäße Verfahren erhaltene Polymethylmethacrylat-Knochenzement ist für die Verwendung in Prepac-Zementiersystemen für die mechanische Fixierung von Gelenkendoprothesen bestimmt. Die Erfindung wird durch nachstehende Beispiele näher erläutert, ohne dass diese jedoch die Erfindung beschränken.

### Beispiel 1: Bestimmung des Aufsaugvermögens des Additivs

Für die Bestimmung des Aufsaugvermögens des Additivs wurden folgende Ausgangsstoffe verwendet:
Methylmethacrylat (Sigma-Aldrich)
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Für die Bestimmung des Aufsaugvermögens der Additive Stärke, Cellulose und Aerosil®380 wurde ein Glasfiltertiegel 1D3 der Firma Schott Mainz verwendet. Es wurde zuerst die Taramasse des Glasfiltertiegels bestimmt. Dann wurden jeweils 3,000 g Additiv und beim Aerosil® 380 1,000g in den Glasfiltertiegel eingewogen. Der Glasfiltertiegel mit dem eingewogenen Additiv wurde auf eine Saugflasche aufgesetzt. Zum Additiv wurden 10 ml Methylmethacrylat gegeben, so dass das Additiv vollständig bedeckt war. Das nicht vom Additiv aufgesaugte Methylmethacrylat lief durch den Glasfiltertiegel nach unten ab. Nach 15 Minuten wurde der Glasfiltertiegel mit dem Additiv und dem aufgesaugten Methylmethacrylat ausgewogen und die Masse des aufgesaugten Methylmethacrylats bestimmt. Die Bestimmung wurde jeweils dreimal wiederholt und der Mittelwert ermittelt. Als Referenz wurde der Glasfiltertiefgel mit Methylmethacrylat ohne zugesetztes Additiv in gleicher Weise behandelt.

| Additiv | Aufsaugvermögen [g Methylmethacrylat/g Additiv] |
|---|---|
| Stärke | 0,7 |
| Cellulose | 1,8 |
| Aerosil® 380 | 9,4 |

### Beispiel 2: Mischen von Zementpulver und Monomerflüssigkeit

Es wurden folgende Ausgangsstoffe für die Herstellung der nachfolgenden Zementpulver 1 bis 11 verwendet:
Methylmethacrylat-methylacrylat-copolymer,
75%iges Dibenzoylperoxid (BPO, phlegmatisiert mit 25 Gew.-% Wasser)
Zirkoniumdioxid,
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Die Komponenten der Zementpulver wurden in 1000 ml Plastikflaschen eingewogen. Danach erfolgte eine Homogenisierung der Zementpulver durch Mischen mit einem TURBULA®-Mischer (Willy A. Bachofen AG). Die Mischzeit betrug 30 Minuten.

**Zusammensetzung der Zementpulver 1-3**

| Zementpulver-Nr. | PMMA-co-MA [g] | ZrO₂ [g] | 75%iges BPO [g] | Stärke [g] |
|---|---|---|---|---|
| 1 | 32,47 | 6,00 | 0,53 | 1,00 |
| 2 | 31,47 | 6,00 | 0,53 | 2,00 |
| 3 | 30,47 | 6,00 | 0,53 | 3,00 |

**Zusammensetzung der Zementpulver 4-6**

| Zementpulver-Nr. | PMMA-co-MA [g] | ZrO₂ [g] | 75%iges BPO [g] | Cellulose [g] |
|---|---|---|---|---|
| 4 | 32,47 | 6,00 | 0,53 | 1,00 |
| 5 | 31,47 | 6,00 | 0,53 | 2,00 |
| 6 | 30,47 | 6,00 | 0,53 | 3,00 |

**Zusammensetzung der Zementpulver 7-11**

| Zementpulver-Nr. | PMMA-co-MA [g] | ZrO₂ [g] | 75%iges BPO [g] | Aerosil® 380 [g] |
|---|---|---|---|---|
| 7 | 33,43 | 6,00 | 0,53 | 0,04 |
| 8 | 33,34 | 6,00 | 0,53 | 0,12 |
| 9 | 33,22 | 6,00 | 0,53 | 0,25 |
| 10 | 32,97 | 6,00 | 0,53 | 0,50 |
| 11 | 32,72 | 6,00 | 0,53 | 0,75 |

Eine transparente, hohlzylinderförmige Kunststoffkartusche mit einem Innendurchmesser von 35 mm wurde mit einem gasdurchlässigen, aber für Zementpulverpartikel undurchlässigen Verschlusskopf für die folgenden Versuche eingesetzt. In die Kartusche wurde 40,00 g Zementpulver gefüllt. Danach wurde der Hohlraum mit dem Zementpulver durch einen für Zementpulverpartikel undurchlässigen, aber für Gase und Flüssigkeiten durchlässigen, axial im Kunststoffrohr verschiebbaren ersten Kolben verschlossen. In den Hohlraum hinter dem ersten Kolben wurde eine Palacos-Ampulle mit 20 ml Monomerflüssigkeit eingelegt. Anschließend wurde ein für Gase, Flüssigkeiten und Zementpulverpartikel undurchlässiger, axial in der Kunststoffkartusche verschiebbarer zweiter Kolben eingesetzt. Sodann wurde das Kunststoffrohr senkrecht gestellt, mit dem Verschlusskopf nach oben. Mit Hilfe einer handbetriebenen, mechanischen Auspressvorrichtung (Palamixgun) wurde dann der zweite Kolben in Richtung des Verschlusskopfs gedrückt. Zuerst brach die Ampulle und die Monomerflüssigkeit und die Splitter wurden in Richtung des zweiten Kolbens gepresst. Die Monomerflüssigkeit wurde dabei durch den zweiten Kolben in das Zementpulver gepresst. Dabei wurde das Zementpulver komplett mit der aufsteigenden Monomerflüssigkeit über eine Strecke von 5,8 cm benetzt. Nach einer Wartezeit von wenigen Sekunden wurde der Verschlusskopf geöffnet und der gebildete Zementteig heraus gepresst.

In einer Variante des Versuchs wurde am Verschlusskopf ein Vakkuumanschluss angesetzt. Es wurden jeweils 40,00 g Zementpulver in die Kartusche gefüllt. Die Kartusche wurde anschließend mit einem für Gase und Flüssigkeiten durchlässigen Kolben verschlossen. Die Kartusche wurde mit dem Kopf nach unten gelagert und Vakuum wurde angelegt. Dann wurden jeweils 20 ml Monomerflüssigkeit auf den für Gase und Flüssigkeiten durchlässigen Kolben gegeben und Vakuum angelegt. Bei allen Zementen wurde die Monomerflüssigkeit über eine Strecke von ca. 4,0 cm durch das Zementpulver gesaugt.

### Beispiel 3: Prüfung nach ISO5833

Für die Herstellung der nachfolgenden Zementpulver wurden folgende Ausgangsstoffe verwendet:
Methylmethacrylat-methylacrylat-copolymer,
75%iges Dibenzoylperoxid (BPO, phlegmatisiert mit 25 Gew.-% Wasser)
Zirkoniumdioxid,
Gentamicinsulfat,
Clindamycinhydrochlorid,
Vancomycinhydrochlorid,
Daptomycin,
Trometamol-Fosfomycin,
Octenidindihydrochlorid
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Als Referenzmaterial wurde der PMMA-Knochenzement PALACOS® LV+G (Lot. 7732, exp. 2017-06) verwendet.

Die Komponenten der Zementpulver wurden in 1000 ml Plastikflaschen eingewogen. Danach erfolgte eine Homogenisierung der Zementpulver durch Mischen mit einem TURBULA-Mischer (Willy A. Bachofen AG). Die Mischzeit betrug 30 Minuten.

**Zusammensetzung der Zementpulver 12-14**

| Zementpulver-Nr. | PMMA-co-MA [g] | ZrO₂ [g] | 75%iges BPO [g] | Stärke [g] |
|---|---|---|---|---|
| 12 | 32,47 | 6,00 | 0,53 | 1,00 |
| 13 | 31,47 | 6,00 | 0,53 | 2,00 |
| 14 | 30,47 | 6,00 | 0,53 | 3,00 |

**Zusammensetzung der Zementpulver 15-17**

| Zementpulver-Nr. | PMMA-co-MA [g] | ZrO₂ [g] | 75%iges BPO [g] | Cellulose [g] |
|---|---|---|---|---|
| 15 | 32,47 | 6,00 | 0,53 | 1,00 |
| 16 | 31,47 | 6,00 | 0,53 | 2,00 |
| 17 | 30,47 | 6,00 | 0,53 | 3,00 |

**Zusammensetzung der Zementpulver 18-22**

| Zementpulver-Nr. | PMMA-co-MA [g] | ZrO₂ [g] | 75%iges BPO [g] | Aerosil® 380 [g] |
|---|---|---|---|---|
| 18 | 33,43 | 6,00 | 0,53 | 0,04 |
| 19 | 33,34 | 6,00 | 0,53 | 0,12 |
| 20 | 33,22 | 6,00 | 0,53 | 0,25 |
| 21 | 32,97 | 6,00 | 0,53 | 0,50 |
| 22 | 32,72 | 6,00 | 0,53 | 0,75 |

**Zusammensetzung der Zementpulver 23-28**

| Zementpulver-Nr. | Wi rkstoff | PMMA-co-MA [g] | ZrO₂ [g] | 75%iges BPO [g] | Aerosil® 380 [g] |
|---|---|---|---|---|---|
| 23 | 1,00 g Gentamicinsulfat | 33,22 | 6,00 | 0,53 | 0,25 |
| 24 | 1,00 g Clindamycinhydrochlorid | 33,22 | 6,00 | 0,53 | 0,25 |
| 25 | 1,00 g Vancomycin-hydrochlorid | 33,22 | 6,00 | 0,53 | 0,25 |
| 26 | 1,00 g Daptomycin | 33,22 | 6,00 | 0,53 | 0,25 |
| 27 | 1,00 g Trometamol-Fosfomycin | 33,22 | 6,00 | 0,53 | 0,25 |
| 28 | 1,00 g Octenidinhydro-chlorid | 33,22 | 6,00 | 0,53 | 0,25 |

Für die Herstellung von Zementteig und der Fertigung von Prüfkörpern wurden 10 ml PALACOS®-Monomerflüssigkeitsampullen (Lot. 5276, exp. 2010-10) der Firma Heraeus Medical GmbH eingesetzt. Die Monomerflüssigkeit einer 10 ml Monomerampulle besteht aus 9,20 g Methylmethacrylat, 0,19 g N,N-Dimethyl-p-toluidin, 10 ppm p-Hydrochinon und 0,2 mg Chlorophyllin (E141).

Es wurden mit den Zementpulvern 12-28 sowie dem Zementpulver Palacos LV+G durch Vermischung mit der Monomerflüssigkeit Zementteigmuster hergestellt. Bei den Zementpulvern 12-22 wurden dazu jeweils 20,0 g Zementpulver mit 10 ml Monomerflüssigkeit vermischt und bei den Zementpulvern 23-28 wurden 20,5 g Zementpulver mit 10 ml Monomerflüssigkeit homogenisiert. Aus dem jeweils gebildeten Zementteig wurden streifenförmige Prüfkörper mit den Abmessungen (75 mm x 10 mm x 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt.

**Ergebnisse der Prüfung der Biegefestigkeit, des Biegemoduls und der Druckfestigkeit gemäß der ISO 5833 der Zementproben 12-28**

| Zementpulver-Nr. | 4-Punkt-Biegefestigkeit [MPa] | Biegemodul [MPa] | Druckfestigkeit [MPa] |
|---|---|---|---|
| 12 | 63,9 ± 1,3 | 2783 ± 87 | 93,0 ± 1,6 |
| 13 | 63,4 ± 1,1 | 2799 ± 80 | 93,7 ± 1,3 |
| 14 | 59,9 ± 1,4 | 2747 ± 103 | 91,6 ± 1,3 |
| 15 | 69,8 ± 1,3 | 2977 ± 96 | 94,4 ± 1,2 |
| 16 | 68,3 ± 2,0 | 2995 ± 135 | 94,4 ± 0,9 |
| 17 | 65,8 ± 1,1 | 2954 ± 57 | 92,5 ± 1,0 |
| 18 | 73,8 ± 1,3 | 3066 ± 59 | 95,6 ± 0,9 |
| 19 | 68,1 ± 2,6 | 2877 ± 20 | 97,7 ± 2,2 |
| 20 | 73,5 ± 1,4 | 2919 ± 78 | 93,2 ± 3,2 |
| 21 | 66,0 ± 2,8 | 2870 ± 60 | 84,5 ± 3,8 |
| 22 | 61,3 ± 3,2 | 2823 ± 3,1 | 89,3 ± 3,6 |
| 23 | 62,2 ± 1,9 | 2871 ± 129 | 94,8 ± 1,4 |
| 24 | 62,5 ± 2,5 | 2788 ± 138 | 92,6 ± 2,2 |
| 25 | 65,8 ± 3,7 | 3004 ± 55 | 94,6 ± 2,6 |
| 26 | 67,5 ± 1,4 | 2970 ± 24 | 93,6 ± 2,0 |
| 27 | 63,8 ± 2,4 | 2881 ± 75 | 91,2 ± 1,0 |
| 28 | 55,6 ± 1,4 | 2829 ± 91 | 89,8 ± 1,9 |
| Referenz Palacos LV+G | 65,2 ± 1,7 | 2851 ± 93 | 91,2 0,9 |

Die ISO5833 schreibt für ausgehärteten Polymethylmethacrylat-Knochenzement eine Biegefestigkeit von mindestens >50 MPa, ein Biegemodul von mindestens >1800 MPa und eine Druckfestigkeit von mindestens >70 MPa vor. Die Ergebnisse der Bestimmung der Biegefestigkeit, des Biegemoduls und der Druckfestigkeit der mit den Zementpulvern 12- 28 hergestellten Zementmuster zeigen, dass die Zementmuster den Anforderungen der ISO5833 hinsichtlich der Biegefestigkeit, des Biegemoduls und der Druckfestigkeit genügen und diese deutlich übertreffen.

## Patentansprüche

1. Verfahren zur Herstellung eines Polymethylmethacrylat-Knochenzements mit den Schritten:
a) Bereitstellen eines Zementpulvers, enthaltend
- mindestens ein partikuläres Polymethylmethacrylat oder Polymethylmethacrylat-Copolymer der Siebfraktion kleiner 100 µm,
- einen Initiator, und
- mindestens ein in Methylmethacrylat unlösliches, partikuläres oder faserförmiges Additiv, wobei das Additiv ein Aufsaugvermögen größer gleich 0,6 g Methylmethacrylat pro Gramm Additiv bei Raumtemperatur besitzt,
b) Bereitstellen einer Monomerlösung, enthaltend
- mindestens ein Methylmethacrylat und
- einen Aktivator
c) In-Kontakt-Bringen des Zementpulvers und der Monomerlösung, **dadurch gekennzeichnet, dass**
das Mischen ohne Anwendung von Scherkräften erfolgt.

2. Verfahren nach Anspruch 1, **dadurch gekennzeichnet,**
**dass** das Additiv kovalent gebundene Hydroxylgruppen an seiner Oberfläche besitzt.

3. Verfahren nach einem der Ansprüchen 1 oder 2, **dadurch gekennzeichnet, dass**
das Additiv ausgewählt ist aus der Gruppe, die aus mikrokristalliner Cellulose, Oxycellulose, Stärke, Titandioxid und Siliziumdioxid besteht.

4. Verfahren nach einem der vorhergehenden Ansprüche, **dadurch gekennzeichnet, dass**
das Additiv eine Partikelgröße der Siebfraktion kleiner 100 µm, bevorzugt der Siebfraktion kleiner 50 µm und ganz besonders bevorzugt von der Siebfraktion kleiner 10 µm hat.

5. Verfahren nach einem der vorhergehenden Ansprüche, **dadurch gekennzeichnet, dass**
das Additiv im Zementpulver in einer Menge von 0,1 bis 2,5 Gew.-% bezogen auf das Gesamtgewicht des Zementpulvers enthalten ist.

## Claims

1. A method for production of a polymethylmethacrylate bone cement comprising the steps of:
a) Providing a cement powder, containing
- at least one particulate polymethylmethacrylate or polymethylmethacrylate copolymer of the sieve fraction smaller than 100 µm;
- an initiator; and
- at least one methylmethacrylate-insoluble, particulate or fibrous additive, whereby the additive possesses an absorption capacity of more than/equal to 0.6 g methylmethacrylate per gram of additive at room temperature;
b) providing a monomer solution, containing
- at least one methylmethacrylate and
- an activator
c) contacting the cement powder and the monomer solution, **characterised in that**
the mixing takes place without shearing forces being applied.

2. The method according to claim 1, **characterised in that**
the additive possesses covalently-bound hydroxyl groups at its surface.

3. The method according to either one of the claims 1 or 2, **characterised in that**
the additive is selected from the group consisting of microcrystalline cellulose, oxycellulose, starch, titanium dioxide, and silicon dioxide.

4. The method according to any one of the preceding claims, **characterised in that**
the additive has a particle size of the sieve fraction smaller than 100 µm, preferably of the sieve fraction smaller than 50 µm, and particularly preferably of the sieve fraction smaller than 10 µm.

5. The method according to any one of the preceding claims, **characterised in that**
the cement powder contains an amount of 0.1 to 2.5% by weight of the additive, relative to the total weight of the cement powder.

## Revendications

1. Procédé de fabrication d'un ciment osseux en polyméthacrylate de méthyle avec les étapes :
a) mise à disposition d'une poudre de ciment contenant
- au moins un polyméthacrylate de méthyle ou copolymère de polyméthacrylate de méthyle particulaire de la fraction granulométrique inférieure à 100 µm,
- un initiateur, et
- au moins un additif insoluble dans le méthacrylate de méthyle, particulaire ou fibreux, dans lequel l'additif possède un pouvoir d'absorption supérieur ou égal à 0,6 g de méthacrylate de méthyle par gramme d'additif à température ambiante,
b) mise à disposition d'une solution monomère contenant
- au moins un méthacrylate de méthyle et
- un activateur,
c) mise en contact de la poudre de ciment et de la solution monomère,
**caractérisé en ce que**
le mélange s'effectue sans application de forces de cisaillement.

2. Procédé selon la revendication 1, **caractérisé en ce**
**que** l'additif possède des groupes hydroxyle liés par covalence à sa surface.

3. Procédé selon une des revendications 1 ou 2, **caractérisé en ce que** l'additif est sélectionné parmi le groupe qui se compose de cellulose microcristalline, oxycellulose, amidon, dioxyde de titane et dioxyde de silicium.

4. Procédé selon une des revendications précédentes, **caractérisé en ce que** l'additif a une taille particulaire de la fraction granulométrique inférieure à 100 µm, de préférence de la fraction granulométrique inférieure à 50 µm et de manière tout particulièrement préférée de la fraction granulométrique inférieure à 10 µm.

5. Procédé selon une des revendications précédentes, **caractérisé en ce que** l'additif est contenu dans la poudre de ciment en une quantité de 0,1 à 2,5 % en poids par rapport au poids total de la poudre de ciment.
